# EUROPEAN PATENT SPECIFICATION

(11) **EP 1 138 336 B1**
(45) Date of publication and mention of the grant of the patent: **10.12.2003**
(21) Application number: 00201189.8
(22) Date of filing: 31.03.2000
(51) Int. Cl.: A61L 27/18, C08G 18/10, C08G 18/42, C08G 18/73

(54) **Biomedical polyurethane-amide, its preparation and use**
Biomedizinisches Polyurethanamid, seine Herstellung und Verwendung
Polyuréthane-amide biomédical, sa préparation et son utilisation

(43) Date of publication of application: 04.10.2001
(73) Proprietor: Polyganics B.V., 9713 GX Groningen (NL)
(72) Inventor: Spaans, Coenraad Jan, 1816 JV Alkmaar (NL); Rienstra, Onno, 9718 LZ Groningen (NL); Pennings, Albert Johan, 3680 Maaseik (BE); de Groot, Jacqueline Hermina, 9351 SR Leek (NL); Belgraver, Willem Vincent, 9715 TK Groningen (NL)
(74) Representative: Prins, Adrianus Willem

(56) References cited:
- WO-A-89/05830
- US-A- 5 100 992
- GROOT DE J H ET AL: "USE OF POROUS POLYURETHANES FOR MENISCAL RECONSTRUCTION AND MENISCAL PROSTHESES" BIOMATERIALS,GB,ELSEVIER SCIENCE PUBLISHERS BV., BARKING, vol. 17, no. 2, 1996, pages 163-173, XP000551706 ISSN: 0142-9612

## Description

Biomedical polymers, such as polyurethanes (including those polyurethanes that also contain amide groups), have been used for a wide range of applications. Examples thereof include nerve guides, meniscal reconstruction materials, artificial skin and artificial veins.

Since sporting activities are increasing significantly in the recent years, there has been a substantial rise in incidence of meniscal injuries. Because the degree of degeneration is proportional to the removed part of the meniscus, nowadays the method of choice is partial meniscectomy in order to preserve as much meniscal tissue as possible. However, repair or replacement of the knee joint meniscus would be more desirable.

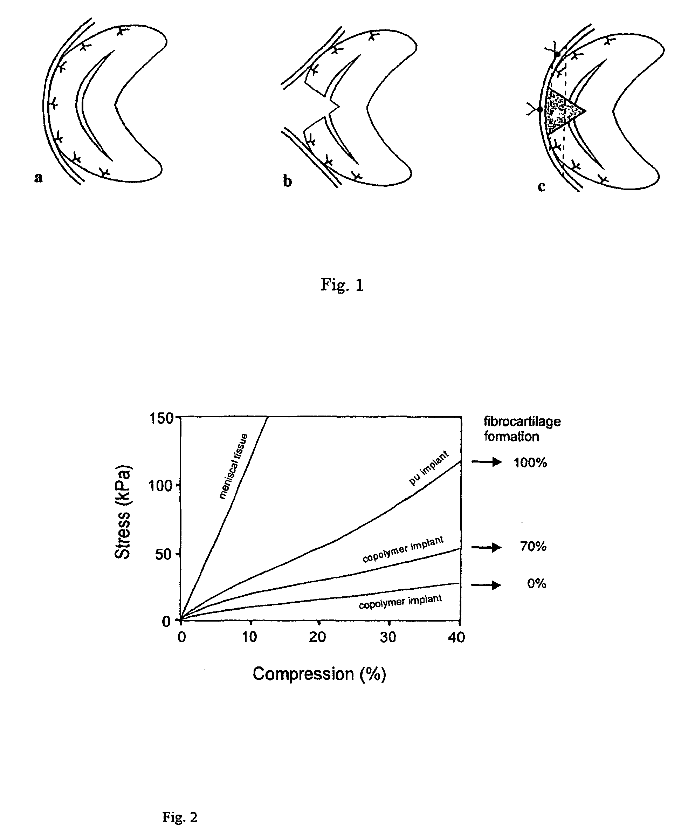

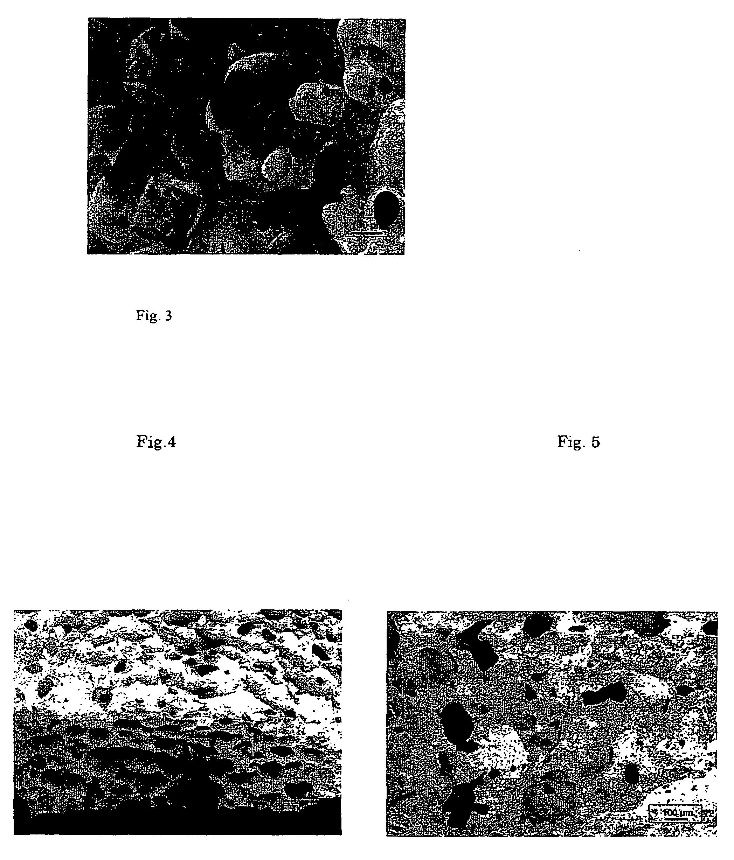

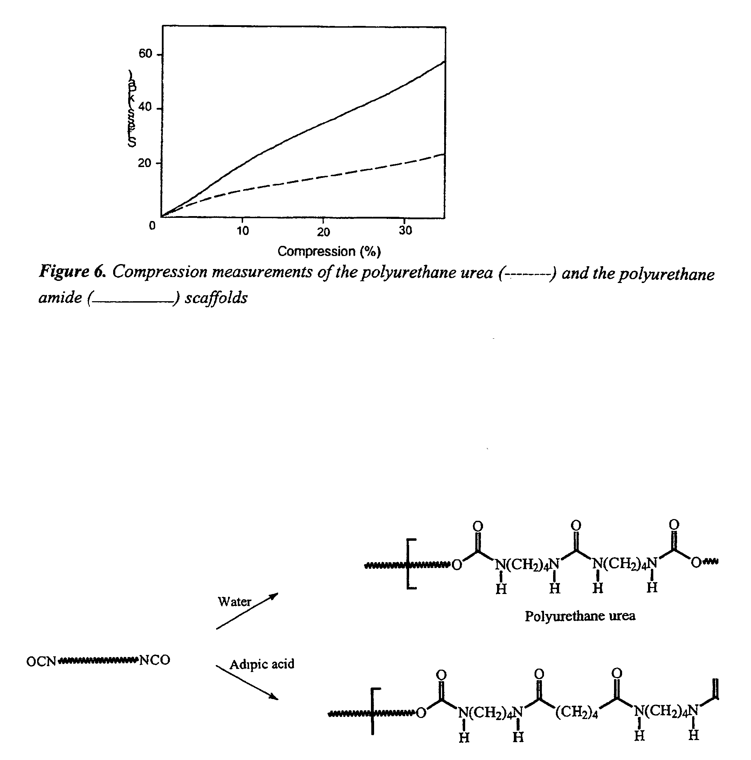

Previously it has been shown that it is possible to repair longitudinal meniscal lesions utilizing porous polymer scaffolds. The meniscus has also already been completely replaced. Lesions in the avascular part (the central two-thirds of the meniscal body) of the meniscus do not heal spontaneously. In order to engineer new meniscal tissue into the lesion, a connection between the lesion and the vascular periphery has been made. The principle of the technique that has been developed for that purpose is shown in figure 1^{a-c}.

The longitudinal meniscal lesion is being connected to the vascular periphery with a second, wedge shaped, access defect. after implantation of the scaffold in the second defect, healing with fibrocartilage was observed.

Earlier, commercially available polyurethanes were used as porous scaffolds. These materials exhibited good mechanical properties but the degradation rate after regeneration of meniscal tissue was rather low. Additionally, these polyurethanes contain methyl diphenyl diisocyanate (MDI) moieties and they might release the corresponding toxic diamine on degradation and sterilization. Other materials that have been used for the same application involved copolymers of ε-caprolactone and L-lactide.⁵ These high molecular weight copolymers exhibit good mechanical properties resulting from crystallizable poly(L-lactide) sequences. Additionally, a quick and complete healing of meniscal lesions was observed, probably caused by the good adhesion between the meniscal tissue and the scaffold. This can be explained in terms of rapid initial degradation resulting into the formation of alcohol and carboxyl functional groups. A second explanation is the aminolysis of the poly(L-lactide/ε-caprolactone) ester bonds by amines present in collagen. This results in covalent bonds between the polymer and the surrounding tissue. Poly(L-Iactide/ε-caprolactone) is known to be extremely susceptible to aminolysis. Drawbacks were, however, that the degradation rate was somewhat too high and that the scaffolds had rather low compression moduli. In figure 2, a plot is shown of the formation of fibrocartilage as a function of the compression of the scaffold.

As can be clearly seen, the compression modulus of the implant should be sufficiently high; more in particular, it should ideally resemble the compression modulus of meniscal tissue. Techniques like freeze-drying frequently allow the fabrication of porous scaffolds with high compression moduli but the solubility of the high molecular weight copolymer is not sufficient. This leads to extremely high porosities and low compression moduli.

Furthermore, it is known that crystallites resulting from the crystallizable poly(L-lactide) sequences may cause a severe inflammatory reaction. A second drawback was that the materials had a rather low resistance to tearing. A high resistance is important in order to prevent sutures from tearing out of the scaffold.

In order to lower the degradation rate of the copolymer and to enhance the resistance to tearing while avoiding long poly(L-lactide) sequences, polyurethanes based on the copolymer were developed. The polymers consisted of 50/50 ε-caprolactone/L-lactide (Mn=2000) soft segments and long, uniform sized, urethane based hard segments. These polyurethanes exhibited reasonably good properties and turned out to be processable into porous scaffolds.

On basis of this previous research it was concluded that a material suitable for meniscal reconstruction should at least fulfill the following criteria:
- Completely degradable into non-toxic products
- High resistance to tear
- Macropores ranging in size from 150-355 µm
- Interconnected pore structure
- Compression modulus of at least 100 kPa
- Good adhesion

The biomedical polyurethanes based on ε-caprolactone/L-lactide seemed to fulfill all these criteria. A drawback, however, was the necessity to use organic solvents (e.g. 1,4-dioxane) for the preparation of the scaffolds.

It is known that residues of organic solvents are likely to remain in these polymers after processing and may damage cells and nearby tissue. Additionally, many active factors are inactivated by exposure to organic solvents. The development of a technique for the preparation of porous materials without the use of organic solvents is therefore highly desirable.

In 1996 and 1998, procedures have been published by Langer and Mooney et al. (Biomaterials **1996** *17* 1417, and Biomed.Mater.Res. **1998** *42* 396) in which polymers are subjected to high carbon dioxide pressure. Porous materials were obtained after quick release of the carbon dioxide. The first materials, however, showed poor mechanical properties and often the pores were closed as a result of skin-formation. Later, this problem was overcome by using additional salt-leaching techniques. This technique, however, proves to be inapplicable for elastomeric polyurethanes because solid polymer/salt discs cannot be obtained.

In Polymer Preprints, **1999**, *40*, 589-590, a procedure is described in which the use of organic solvents is avoided. The procedure involved the reaction of an isocyanate end-capped ε-caprolactone/L-lactide based prepolymer with water, analogous to the procedure published by Ikada et al, *J.Biomed.Mater.Res,***1991**, 25, 1481. Chain extension with water is a well-known method to obtain polyurethane ureas. During the reaction carbon dioxide is formed, resulting into a porous material. The macropores showed good interconnectivity.

In WO-A 9964491 the production of polyurethane ureas is described using well-defined hard blocks to obtain an improved balance of the mechanical properties.

In both systems it turned out to be difficult to obtain good mechanical properties, probably caused by transesterification, aminolysis and hydrolysis reactions of the polyester soft segment with water. Furthermore, although salt crystals were added, regulation of the pore size and pore uniformity remained difficult. An additional problem was the excess of carbon dioxide produced in the foaming reaction.

Accordingly it is an object of the present invention to provide macroporous biomedical polyurethane-amide based materials, having good mechanical properties, that can be prepared by an easy in-situ foam generating process, requiring a relatively short reaction time, without the use of solvents.

The invention is directed to novel biomedical polyurethanes, suitable for implants, not having the disadvantages discussed above.

In a first aspect the invention is directed to a novel in situ produced macroporous biomedical polyurethane-amide material based on chain extended isocyanate terminated polyester prepolymer units, wherein the said chain extension has been done with at least one dicarboxylic acid or a hydroxy-carboxylic acid.

In another aspect the invention is directed to a novel in situ produced macroporous biomedical polyurethane-amide material based on chain extended prepolymer units of biocompatible soft polyester segments and on hard urethane-amide segments, said material having a compression modulus of at least 100 kPa and a pore size distribution less than 10 vol.% of pores having a pore size > 450 µm.

Surprisingly it has been found that it is possible to provide an in-situ produced macroporous biomedical polyurethane-amide that meets the requirements set forth above.

An important aspect of the material is the specific chain extension using dicarboxylic or hydroxy carboxylic acids, with in-situ generation of foam, while preventing the use of solvents of water.

Further embodiments have been given in the claims, as well as in the subsequent description and examples.

The products of the present invention show a good balance between the properties necessary for use thereof in biomedical applications, such as good modulus, tear strength and compression modulus. It has been found possible to produce porous implants by the in-situ generation of gas for foaming, optionally combined with salt-leaching, resulting in a material having macropores in the range of 50 µm to 450 µm, more preferred between 150 and 375 µm (defined by electron microscopy). For meniscus repair, preferably, at least 50 % by volume of the pores have a pore size in the range of 50 µm to 450 µm. and also preferred in a volume of at least 30 % of pores with a size between 150 and 375 µm.

As has been indicated above, the conventional methods of producing polyurethanes (endcapping with diisocyanate and chain extending with water) may result in transesterification, hydrolysis and aminolysis, with the consequence that the material has insufficiently balanced properties. More in particular branching and cross-linking occur, resulting in loss of phase separation. The consequence thereof is that the mechanical properties deteriorate to a level below that which is acceptable for numerous biomedical applications.

The present invention is applicable to various types of implants in the human or animal body, such as for meniscal reconstruction, but also for other types of implants, including artificial skin, nerve guides, vascular protheses and medical devices such as wound dressings.

The nature of the application determines the level of biodegradability, which is mainly determined by the nature of the polyester prepolymer. Generally a material is considered biodegradable in case it degrades into non-toxic components within a reasonable period of time. Degradation can be determined by following the mechanical properties of the material as function of the time. For meniscal repair material it is preferred that the mechanical properties as indicated earlier, are maintained for a certain period of time, for example at least three months, following which they start to drop.

As has been indicated above, the polyurethane of the invention comprises in the most general form chain extended isocyanate terminated polyester prepolymer units, the said chain extension being based on least one dicarboxylic acid or a hydroxy-carboxylic acid, resulting in urethane-amide segments. The nature of these urethane-amide segments is important, especially with respect to obtaining good phase separation and accordingly well-balanced properties.

Phase separation may occur between a soft and a hard phase, the soft phase being composed of the polyester material, optionally in combination with an additional polyether polyol, and the hard phase being composed of the chain extender and diisocyanate endcapping moieties.

The polyester to be used in accordance with the invention will preferably be linear, more in particular be a random copolyester, and will have reactive endgroups. These endgroups may be hydroxyl or carboxyl. It is preferred to have a dihydroxy terminated copolyester, but hydroxy-carboxyl or dicarboxyl terminated copolyesters can also be used. The nature of the endgroups is determined by the type of comonomers, the amounts thereof, the type of starter (if used), and the reaction conditions.

Suitable monomers for the polyester are the cyclic monomers that can be polymerised under ring-opening polymerisation conditions. Examples are lactides, glycolides, trimethylene carbonate and/or ε-caprolacton. Preferred are lactide (D, L, D-L, meso) and ε-caprolacton. More in particular a linear random copolyester having about equimolar amounts of ε-caprolacton and L-Lactide is preferred. Other possibilities include polyesters based on succinic acid and ethylene glycol or 1,4-butanediol, or on (co)polyesters of lactic acid. In case the polyester has to be linear, it can be prepared using a difunctional component (diol) as starter, but in case a three or higher functional polyol is used, star shaped polyesters may be obtained. By careful selection of the type of materials, the properties of the final material, more in particular relating to the degradability, can be determined.

The conditions for preparing the polyesters are those known in the art.

The (linear random co)polyester is preferably endcapped by diisocyanate. The type of diisocyanate used herein is of some importance. In case the material is used for biodegradable implants and the like, the degradation products should be non-toxic. Examples of suitable isocyanates are IPDI, lysine-diisocyanaat, the terephtalic type diisocyanates, and aliphatic diisocyanates, more in particular 1,4-butane diisocyanate(BDI). The nature of the diisocyanate is also important for the phase separation, in combination with the type of chain extender. It is also possible to endcap with isocyanate terminated materials, such as small blocks of diisocyanate terminated diol (BDI-butanediol-BDI).

It is possible to combine the polyester with a polyol, such as a polyether-polyol, in order to modify the properties thereof (biodegrability, phase separation, hydrophilicity, mechanical properties). This polyol may either be included in the polyester itself, or may be mixed with the polyester, prior to endcapping. Preferably low molecular weight polyols are used (Mw from 500 to 3000, based on ethylene and/or propylene glycol).

The prepolymer is subsequently reacted with the chain extender (dicarboxylic acid or hydroxy carboxylic acid), optionally in combination with a polyol, to yield a polyurethane amide (in the theoretical form). It is to be noted that various side reactions may occur. It is possible to include a polyol, such as a polyether polyol or other diol, in the system during the chain extension. The same polyols as discussed above may be used, but also hydroxyl terminated polyester prepolymers having a low molecular weight (Mw: 750 to 3000). In such case, the amount of CO₂ generated is reduced. This is a good method of regulating the pore volume. The ratio of hydroxyl to carboxyl groups in the chain extension may vary widely, depending on the required porosity and other properties. This ratio may vary between 0 and 3.

The chain extender to be used is either a dicarboxylic acid or a hydroxycarboxylic acid. An important consideration in the selection thereof is the nature of the material in which it is used, biomedical materials. This means that the residues of the chain extender must be biocompatible. Examples of suitable chain extenders are the dicarboxylic acids, such as adipic acid, succinic acid, and also urethane addition products having two terminal carboxylic acid groups (XYX, wherein Y designates diisocyanate and X a carboxylic acid containing moiety, such as a dicarboxylic acid). Also hydrocarboxylic acids may be used, having the formula HO-(CH₂)ₙ-COOH, n being an even number. Other examples are lactic acid and lactoyl lactic acid.

The chain extension may further be carried out in the presence of a catalytic amount of a catalyst, such as Sn-octoate.

An advantage of the chain extension using carboxylic acids, is that less branching and cross-linking occurs. Secondly, organic materials have a lower reactivity than water which makes it possible to obtain more homogeneous reaction mixtures and thus more homogeneous foams. Furthermore, carboxylic acids are less nucleophilic resulting into less transesterification reactions and thus more uniform-sized hard segments. DSC measurements showed a lower Tg compared to the water chain-extended materials, indicative for better micro-phase separation. Furthermore, a single sharp melting endotherm was observed.

This process in situ generates porous materials by the formation of CO₂ in the reaction of the isocyanate and the carboxylic acid. The formation of the macropores can further be regulated and improved by mixing the prepolymer with salt crystals of the required pore size. After leaching the salt from the material a porous polyurethane amide has been obtained, whereby part of the pores are provided by the salt and part by the CO₂ generated in the reaction of the prepolymer with the chain extender. According to a preferred embodiment the chain extender is mixed with the prepolymer as a fine powder, thereby obviating the need to use solvents or water in the system. This has the additional advantage that the powder acts as nucleant for the pore forming. Especially with adipic acid, this is a good option. It is also possible to add additional nucleants, such as the various solid carbonate salts. Commonly applied nucleants include potassium carbonate, calcium carbonate, sodium bicarbonate and citric acid.

At this stage it is also possible to apply ultrasonic treatment to improve the generation of pores. High amplitude ultrasonic waves prevent small cells from coalescing into larger cells, as may happen at low amplitudes or in the absence of ultrasonic waves..

When ultrasonic waves were applied, an enhancement in cell regularity was observed. Furthermore, chain-extension with acid instead of water resulted in a more regular pore-structure and smaller pores. An explanation for this effect might be that the acid serves a dual role in the reaction system. During the reaction at the acid surface, the carboxylic acid is not only consumed as reactant but it also works as nucleating agent. When the acid is finely powdered prior to use, the pore size decreases further. Thus, it can be assumed that indeed the acid solid particles play an important role in the pore-size of the resulting foams. In figures x and y foams with finely powdered adipic acid are shown.

The reactions between the various components are carried out under the conditions known to be suitable for the preparation of polyurethanes and polyuerthane-amides from diisocyanates.

These processes all result in a useful biomedical polyurethane-amides, having the advantageous properties cited above.

Although this type of bulk polymerization is a routine process in industry (e.g. for fabrication of insulation materials) examples of fabrication of porous biomaterials using this technique are rare. Often, the regular structures are obtained by the use of various additives. For obvious reasons, the possibility of using additives is limited or impossible in the case of biomedical foams.

According to a preferred embodiment a porous polyurethane amide was synthesized in a 2-step polymerization process. In the first step, a hydroxyl-terminated prepolymer (Mn=2000) of 50/50 ε-caprolactone/L-lactide was end-capped with a 6-fold excess of 1,4-butanediisocyanate. The excess of diisocyanate was used in order to avoid the formation of prepolymer dimers, trimers etc. It has been shown that prepolymer dimers and trimers result in non-uniform hard segments, lower molecular weights and inferior mechanical properties.

After removal of the excess of diisocyanate, the BDI-terminated prepolymer was mixed with salt crystals (150-355 µm), surfactant (dodecyl sulfate) and hydroxyl-terminated copolymer (Mn=1000). The salt crystals were added in order to induce more macro-porosity. Surfactant was added in order to lower the surface tension of the system and thus to decrease the pore size. An additional effect is that the resulting pore structure will be more regular. The low molecular weight hydroxyl terminated copolymer was added in order to decrease the amount of carbon dioxide of the foaming reaction.

After addition of the chain extender, the reaction mixture was stirred and the homogeneous mixture was allowed to react at 80 °C. After 8 hours of reaction and washing out the salt crystals, a cellular product was obtained.

As a comparison also the same system was prepared, using water as a chain extender, resulting in a polyurethane urea. The polyurethane amides and polyurethane urea are formed according to scheme 1, as attached.

It should be noted that in the case of the polyurethane urea, hydrolysis, cross-linking and branching reactions have occurred, resulting in poorly defined hard segments. This was confirmed by the fact that the polymer did not dissolve in polar solvents but only showed swelling. Furthermore, due to the addition of hydroxyl-terminated prepolymer, additional urethane moieties are present.

The invention is now elucidated on the basis of the examples.

### Experimental

### Materials and eqpuipment

Prepolymer synthesis was performed under nitrogen atmosphere in flame-dried glassware. L-lactide (Hycail B.V., The Netherlands) was recrystallized from sodium dried toluene, ε-caprolactone (Acros Organics, Belgium) was distilled under reduced nitrogen pressure from CaH₂. 1,4-Butanediol (BDO, Acros Organics, Belgium) was distilled under reduced nitrogen pressure from 4Å molecular sieves and 1,4-butanediisocyanate (BDI, DSM Research, The Netherlands) was distilled under reduced nitrogen pressure prior to use. Adipic acid (Acros Organics, Belgium) was used as received from the supplier.

A Branson sonifier 250, operated at 20 kHz and equipped with a standard horn-tip was used to generate ultrasonic waves.

### Prepolymer synthesis

Hydroxyl-terminated prepolymers (Mn=2000 and 1000) of 50/50 ε-caprolactone/L-lactide were prepared using 1,4-butanediol as initiator and stannous octoate as a catalyst. After reaction at 120 °C for 20 hours, ¹H NMR showed complete conversion.

The hydroxyl-terminated prepolymer (Mn=2000) was reacted with a six-fold excess of 1,4-butanediisocyanate at 80 °C for 4 hours. After the reaction was complete, the excess of diisocyanate was distilled off under reduced pressure (0.005 mbar) at 80-90 °C using a Kugelrohr apparatus resulting into a butanediisocyanate-terminated prepolymer.

### Preparation of the porous polyurethane amides and polyurethane ureas

The macro-diisocyanate (3.0 g) was heated to 80 °C and mixed with NaCl crystals (3.0 g) varying in size from 150-355 µm. Subsequently, a small amount of (solid) sodium dodecylsulfate (∼0.1 g), hydroxyl terminated copolymer (Mn=1000, viscous liquid) and finely powdered adipic acid (1.5 eq.) or water were added. Stirring was performed with a mechanical stirrer at a frequency of 2000 rpm. The vial with the homogeneous reaction mixture was immersed in an oil bath at 80 °C for 8 hours. In some cases, ultrasonic waves (20.000 Hz, high amplitude, output 90 W) were applied; the temperature was then allowed to rise to 95 °C. After reaction, the resulting foam/salt mixture was allowed to cool to room temperature, the vial was broken and the salt was washed out with water. After complete removal of salt the foams were dried under reduced pressure.

### Characterization

Calorimeter studies were carried out with a Perkin Elmer DSC 7 calorimeter. The scanning rate was 10°C per minute.
¹H-NMR (200 MHz) was used to characterize the materials.

### Compression measurements

Compression curves were determined at room temperature using an Instron (4301) tensile tester equipped with a 100N load-cell at a cross-head speed of 12 mm/min. Cylindrical specimens were cut out of the foams under liquid nitrogen. Compression was performed between parallel plates using fully dried samples.

### Electron microscopy

A Jeol 6320 F Field Emission Scanning Electron Microscope (FESEM) was used for studying the pore structure of the porous materials. It was operated at a working distance of 11 mm, an acceleration voltage of 5kV and a beam current of 1 x 10 ⁻¹⁰ A. The specimens were made conductive with a 3 nm layer of gold using a Cressington Rotating Magnetron Sputter Coater operated at a working distance of 150 mm and a current of 20 mA.

### Results

As can be seen from figures x and y, foams with a rather regular pore structure have been obtained. Nearly all pores range in size from 100-380 µm. The distribution in pore-size does not limit the applicability as meniscal reconstruction material. Importantly, the pores are interconnected, as can be seen from the SEM micrograph and the fact that all the salt crystals could be washed out with water. The latter was confirmed by SEM micrographs of various cross-sections. The porosity of the scaffolds lies around 70% which was determined by weight and volume, assuming a polymer density of 1.1 g/ml.

The resulting polyurethane urea and polyurethane amide foams have been subjected to compression measurements. As already mentioned, the compression modulus is an important parameter in the formation of fibrocartilage and thus in the repair of meniscal lesions. In figure 6, the compression behavior of the foams obtained by chain extension with water and adipic acid are shown. The porosity of the polyurethane urea foam and the polyurethane amide foam lies around 70-80%

Although the porosities are nearly equal, the polyurethane amide foam has a higher compression modulus than the polyurethane urea foam. This is probably caused by hydrolysis during the polyurethane urea foaming reaction. The resulting hard segments are not of uniform length giving rise to phase mixing. This prevents an effective crystallization like in the case of the polyurethane amide. In the case of the polyurethane amide, hard segments of uniform size have been obtained and the polymer has a more phase separated morphology resulting into a higher compression modulus.

The compression modulus of 150 kPa of the polyurethane amide is sufficiently high to serve as meniscal reconstruction material. Eventually, lowering the porosity of the scaffold can enhance the compression modulus. The interconnectivity might be increased by further lowering the pore size.

## Claims

1. In situ produced macroporous biomedical polyurethane-amide material based on chain extended isocyanate terminated polyester prepolymer units, wherein the said chain extension has been done with at least one dicarboxylic acid or a hydroxy-carboxylic acid.

2. Polyurethane-amide according to claim 1, wherein the material has a pore structure, wherein the amount of pores having a pore size of >450 µm is less than 10% by volume.

3. Polyurethane-amide according to claim 1 or 2, wherein the material has an open cell structure.

4. Polyurethane-amide according to claim 1-3, wherein the said prepolymer is a prepolymer of soft polyester segments, having a glass transition temperature below 40°C, said prepolymer further optionally containing polyether-polyol segments.

5. Polyurethane-amide according to claim 1-4, wherein the material shows phase separation into hard an soft phases.

6. Polyurethane-amide according to claim 1-5, wherein the polyester is based on a polyester prepared by ringopening polymerisation, preferably a random copolyester.

7. Polyurethane-amide according to claim 6, wherein the random copolyester is a copolyester of lactide, glycolide, trimethylene carbonate and/or ε-caprolacton.

8. Polyurethane-amide according to claim 1-7, further comprising an additional diol segment.

9. Polyurethane-amide according to claim 8, wherein the said additional diol segment is a polyether or a polyester segment.

10. Polyurethane-amide according to claim 8 or 9, wherein the said diol segment is incorporated in the material during the reaction of the prepolymer with the chain extender.

11. Polyurethane-amide according to claim 1-10, based on a copolyester of lactide and ε-caprolacton containing 5 to 95, preferably 40-60 % of units of lactide and 5 to 95, preferably 40-60 % of units of ε-caprolacton, based on number.

12. In situ produced macroporous biomedical polyurethane-amide material based on chain extended prepolymer units of biocompatible soft polyester segments and on hard urethane-amide segments, said material having a compression modulus of at least 100 kPa and a pore size distribution less than 10 vol.% of pores having a pore size > 450 µm.

13. Macroporous biomedical polyurethane-amide according to claim 12, showing phase separation between soft and hard segments.

14. Macroporous biomedical polyurethane-amide according to claim 12 or 13, having an open cell structure.

15. Macroporous biomedical polyurethane-amide according to claim 12-14, said material being biodegradable.

16. Process for the preparation of a macroporous biomedical polyurethane-amide according to claim 1-15, said process being solvent free and comprising preparing an isocyanate terminated polyester prepolymer, mixing the prepolymer with at least one chain extender selected from the group of dicarboxylic acids and hydroxycarboxylic acids, reacting the mixture to produce the macroporous biomedical polyurethane.

17. Process according to claim 16, wherein the said chain extender is adipic acid.

18. Process according to claim 16 or 17, wherein the prepolymer is mixed with salt crystals of a required particle size to assist in the generation of suitable pores, and leaching out the salt crystals after the chain extension has been completed.

19. Process according to claim 16-18, wherein the chain extension is performed in the additional presence of a diol.

20. Process according to claim 16-19, wherein a nucleant is present during chain extension, said nucleant preferably being either powdered adipic acid, also acting as chain extender, or a powdered inert material.

21. Process according to claim 16-20, wherein during the chain extension the reaction mixture is treated ultrasonically.

22. Process according to claim 16-21, wherein the reaction mixture also contains a surfactant.

23. Macroporous biomedical polyurethane-amide material according to claim 1-15, or produced in accordance with the process of claim 16-22, for use in human or veterinary surgery, as implant or repair material.

24. Implant or reconstruction material in human or veterinary surgery based on the biomedical polyurethane-amidess according to claim 1-15, or produced in accordance with the process of claim 16-22.

25. Porous scaffold for repairing meniscal lesion, comprising the macroporous biomedical polyurethane-amide according to claim 1-15, or produced in accordance with the process of claim 16-22.

## Patentansprüche

1. In situ hergestelltes makroporöses biomedizinisches Polyurethanamid-Material auf der Basis von kettenverlängerten isocyanatterminierten Polyester-Prepolymereinheiten, wobei die Kettenverlängerung mit wenigstens einer Dicarbonsäure oder einer Hydroxycarbonsäure erfolgt ist.

2. Polyurethanamid gemäß Anspruch 1, wobei das Material eine Porenstruktur hat, wobei die Menge der Poren mit einer Porengröße von > 450 µm kleiner als 10 Vol.-% ist.

3. Polyurethanamid gemäß Anspruch 1 oder 2, wobei das Material eine offenzellige Struktur hat.

4. Polyurethanamid gemäß Anspruch 1 bis 3, wobei das Prepolymer ein Prepolymer aus weichen Polyestersegmenten mit einer Glasübergangstemperatur von unter 40 °C ist, wobei das Prepolymer gegebenenfalls weiterhin Polyether-Polyol-Segmente enthält.

5. Polyurethanamid gemäß Anspruch 1 bis 4, wobei das Material eine Phasentrennung in harte und weiche Phasen zeigt.

6. Polyurethanamid gemäß Anspruch 1 bis 5, wobei der Polyester auf einem Polyester, der durch Ringöffnungspolymerisation hergestellt wird, vorzugsweise einem statistischen Copolyester, beruht.

7. Polyurethanamid gemäß Anspruch 6, wobei der statistische Copolyester ein Copolyester von Lactid, Glycolid, Trimethylencarbonat und/oder ε-Caprolacton ist.

8. Polyurethanamid gemäß Anspruch 1 bis 7, das weiterhin ein zusätzliches Diolsegment umfasst.

9. Polyurethanamid gemäß Anspruch 8, wobei das zusätzliche Diolsegment ein Polyether- oder Polyestersegment ist.

10. Polyurethanamid gemäß Anspruch 8 oder 9, wobei das Diolsegment während der Reaktion des Prepolymers mit dem Kettenverlängerer in das Material eingebaut wird.

11. Polyurethanamid gemäß Anspruch 1 bis 10, basierend auf einem Copolyester von Lactid und ε-Caprolacton, der 5 bis 95, vorzugsweise 40 bis 60% Lactideinheiten und 5 bis 95, vorzugsweise 40 bis 60 ε-Caprolacton-Einheiten enthält, bezogen auf deren Anzahl.

12. In situ hergestelltes makroporöses biomedizinisches Polyurethanamid-Material auf der Basis von kettenverlängerten Prepolymereinheiten aus biokompatiblen weichen Polyestersegmenten und harten Urethanamid-Segmenten, wobei das Material einen Druckmodul von wenigstens 100 kPa und eine Porengrößeverteilung, bei der weniger als 10 Vol.-% der Poren eine Porengröße von > 450 µm haben, aufweist.

13. Makroporöses biomedizinisches Polyurethanamid gemäß Anspruch 12, das eine Phasentrennung zwischen weichen und harten Segmenten zeigt.

14. Makroporöses biomedizinisches Polyurethanamid gemäß Anspruch 12 oder 13, das eine offenzellige Struktur aufweist.

15. Makroporöses biomedizinisches Polyurethanamid gemäß Anspruch 12 bis 14, wobei das Material biologisch abbaubar ist.

16. Verfahren zur Herstellung eines makroporösen biomedizinischen Polyurethanamids gemäß Anspruch 1 bis 15, wobei das Verfahren lösungsmittelfrei ist und folgendes umfasst: die Herstellung eines isocyanatterminierten Polyester-Prepolymers, das Mischen des Prepolymers mit wenigstens einem Kettenverlängerer, der aus der Gruppe der Dicarbonsäuren und Hydroxycarbonsäuren ausgewählt ist, und das Umsetzen des Gemischs unter Bildung des makroporösen biomedizinischen Pofyurethans.

17. Verfahren gemäß Anspruch 16, wobei es sich bei dem Kettenverlängerer um Adipinsäure handelt.

18. Verfahren gemäß Anspruch 16 oder 17, wobei das Prepolymer mit Salzkristallen einer erforderlichen Teilchengröße gemischt wird, um die Entstehung geeigneter Poren zu unterstützen, und die Salzkristalle ausgelaugt werden, nachdem die Kettenverlängerung beendet ist.

19. Verfahren gemäß Anspruch 16 bis 18, wobei die Kettenverlängerung zusätzlich in Gegenwart eines Diols durchgeführt wird.

20. Verfahren gemäß Anspruch 16 bis 19, wobei während der Kettenverlängerung ein Keimbildner vorhanden ist, wobei es sich bei dem Keimbildner vorzugsweise entweder um gepulverte Adipinsäure, die auch als Kettenverlängerer wirkt, oder um ein gepulvertes inertes Material handelt.

21. Verfahren gemäß Anspruch 16 bis 20, wobei das Reaktionsgemisch während der Kettenverlängerung mit Ultraschall behandelt wird.

22. Verfahren gemäß Anspruch 16 bis 21, wobei das Reaktionsgemisch auch ein Tensid enthält.

23. Makroporöses biomedizinisches Polyurethanamid-Material gemäß Anspruch 1 bis 15 oder hergestellt gemäß dem Verfahren von Anspruch 16 bis 22 zur Verwendung in Human- oder Veterinärchirurgie als Implantat oder Reparaturmaterial.

24. Implantat oder Rekonstruktionsmaterial in der Human- oder Veterinärchirurgie auf der Basis der biomedizinischen Polyurethanamide gemäß Anspruch 1 bis 15 oder hergestellt gemäß dem Verfahren von Anspruch 16 bis 22.

25. Poröses Gerüst zum Reparieren von Meniskusläsionen, umfassend das makroporöse biomedizinische Polyurethanamid gemäß Anspruch 1 bis 15 oder hergestellt gemäß dem Verfahren von Anspruch 16 bis 22.

## Revendications

1. Matériau biomédical polyuréthane-amide macroporeux produit *in situ* à base d'unités de prépolymère polyester terminé par isocyanate à chaîne étendue, la dite extension de chaîne étant réalisée avec au moins un acide dicarboxylique ou un acide hydroxycarboxylique.

2. Polyuréthane-amide selon la revendication 1, le matériau ayant une structure à pores, où la quantité de pores ayant une dimension > 450 µm est inférieure à 10 % en volume.

3. Polyuréthane-amide selon la revendication 1 ou 2, le matériau ayant une structure à alvéoles ouverts.

4. Polyuréthane-amide selon les revendications 1 à 3, où ledit prépolymère est un prépolymère de segments polyesters souples, ayant une température de transition vitreuse inférieure à 40°C, ledit prépolymère contenant en outre, de manière optionnelle, des segments polyéther-polyol.

5. Polyuréthane-amide selon les revendications 1 à 4, le matériau présentant une séparation de phases en phases rigide et souple.

6. Polyuréthane-amide selon les revendications 1 à 5, où le polyester est basé sur un polyester préparé par polymérisation avec ouverture de cycle, de préférence un copolyester statistique.

7. Polyuréthane-amide selon la revendication 6, où le copolyester statistique est un copolyester de lactide, de glycolide, de triméthylènecarbonate et/ou d'ε-caprolactone.

8. Polyuréthane-amide selon les revendications 1 à 7, comprenant en outre un segment diol supplémentaire.

9. Polyuréthane-amide selon la revendication 8, dans lequel ledit segment diol supplémentaire est un segment polyéther ou polyester.

10. Polyuréthane-amide selon la revendication 8 ou 9, dans lequel ledit segment diol est incorporé dans le matériau pendant la réaction du prépolymère avec l'agent d'extension de chaîne.

11. Polyuréthane-amide selon les revendications 1 à 10, à base d'un copolyester de lactide et d'ε-caprolactone, contenant 5 à 95 %, de préférence 40 à 60 %, d'unités lactides et 5 à 95 %, de préférence 40 à 60 %, d'unités ε-caprolactone, exprimées en nombre.

12. Matériau biomédical polyuréthane-amide macroporeux produit *in situ* à base d'unités de prépolymères à chaîne étendue de segments polyesters souples biocompatibles et de segments uréthane-amide rigides, ledit matériau ayant un module de compression d'au moins 100 kPa et une distribution des dimensions des pores telle que moins de 10 % de pores en volume ont une dimension > 450 µm.

13. Polyuréthane-amide macroporeux biomédical selon la revendication 12, présentant une séparation de phases entre segments rigides et souples.

14. Polyuréthane-amide macroporeux biomédical selon la revendication 12 ou 13, ayant une structure à alvéoles ouverts.

15. Polyuréthane-amide macroporeux biomédical selon les revendications 12 à 14, ledit matériau étant biodégradable.

16. Procédé pour la préparation d'un polyuréthane-amide macroporeux biomédical selon les revendications 1 à 15, ledit procédé étant sans solvant et comprenant la préparation d'un prépolymère polyester terminé par isocyanate, le mélange du prépolymère avec au moins un agent d'extension de chaîne choisi dans le groupe constitué par les acides dicarboxyliques et les acides hydroxycarboxyliques, la réaction du mélange pour produire le polyuréthane macroporeux biomédical.

17. Procédé selon la revendication 16, dans lequel ledit agent d'extension de la chaîne est l'acide adipique.

18. Procédé selon la revendication 16 ou 17, dans lequel le prépolymère est mélangé avec des cristaux de sel dont les particules ont une dimension requise pour faciliter la génération de pores appropriés, et l'élimination des cristaux de sel par lessivage après la fin de l'extension de chaîne.

19. Procédé selon les revendications 16 à 18, dans lequel l'extension de la chaîne est effectuée en présence d'un diol additionnel.

20. Procédé selon les revendications 16 à 19, dans lequel un agent de nucléation est présent pendant l'extension de la chaîne, ledit agent de nucléation étant de préférence de l'acide adipique en poudre, agissant également comme agent d'extension de chaîne, ou une matière inerte en poudre.

21. Procédé selon les revendications 16 à 20, dans lequel, pendant l'extension de la chaîne, le mélange réactionnel est traité par des ultrasons.

22. Procédé selon les revendications 16 à 21, dans lequel le mélange réactionnel contient également un tensioactif.

23. Matériau biomédical polyuréthane-amide macroporeux selon les revendications 1 à 15, ou produit selon le procédé des revendications 16 à 22, pour son utilisation en chirurgie humaine ou vétérinaire, en tant que matériau pour implant ou pour réparation.

24. Matériau pour implant ou reconstruction en chirurgie humaine ou vétérinaire à base des polyuréthane-amides biomédicaux selon les revendications 1 à 15 ou produits selon le procédé des revendications 16 à 22.

25. Echafaudage poreux pour la réparation d'une lésion du ménisque, comprenant le polyuréthane-amide macroporeux biomédical selon les revendications 1 à 15 ou produit selon le procédé des revendications 16 à 22.
